# EUROPÄISCHE PATENTSCHRIFT

(11) **EP 1 121 902 B1**
(45) Veröffentlichungstag und Bekanntmachung des Hinweises auf die Patenterteilung: **22.09.2004**
(21) Anmeldenummer: 01100933.9
(22) Anmeldetag: 17.01.2001
(51) Int. Cl.: A61B 17/70

(54) **Pedikelschraube**
Pedicle screw
Vis pédiculaire

(30) Priorität: 07.02.2000 DE 10005385
(43) Veröffentlichungstag der Anmeldung: 08.08.2001
(73) Patentinhaber: Ulrich GmbH & Co. KG, 89081 Ulm (DE)
(72) Erfinder: Schäffler-Wachter, Martin, 89231 Neu-Ulm (DE); Schönhöffer, Helmut, 89155 Erbach (DE)
(74) Vertreter: Hentrich, Swen Dipl.-Phys. Dr.

(56) Entgegenhaltungen:
- EP-A- 0 346 521
- WO-A-99/65415
- DE-A- 4 107 480
- DE-A- 19 732 188

## Beschreibung

Die Erfindung betrifft eine Pedikelschraube für Implantate zur Korrektur und Stabilisierung der Wirbelsäule, mit einem an dem axialen Ende eines Gewindeschafts angeordneten Kopfteil, an dem ein Bügelteil anschließbar ist, das U-förmig mit einer Grundplatte und zwei Seitenschenkeln gebildet ist und eine Aufnahme für einen am Kopfteil fixierbaren Stab aufweist, wobei das Kopfteil ein Rundstück aufweist.

Pedikelschrauben sind beispielsweise aus der DE 41 07 480 A1 bekannt. Bei diesen bekannten Pedikelschrauben übergreift das Bügelteil das Kopfteil mit seitlichen Schenkelteilen, in denen parallel zur Längsrichtung des Stabes verlaufende, einseitig offene Nuten ausgebildet sind, in die am Kopfteil vorgesehene und entsprechend verlaufende Leisten formschlüssig eingreifen, wobei zur Vermeidung von Abspreizungen die Leisten und Nuten an ihren aneinanderliegenden Schenkelteilen schwalbenschwanzartige Hinterschneidungen aufweisen. Diese Pedikelschrauben haben sich in der Praxis gut bewährt, da es mit diesen möglich ist, eine stabile und dauerhafte Fixierung der Lage des Stabes zu ermöglichen. Allerdings ist für die Plazierung und gegenseitige Fixierung von der Pedikelschraube und dem Stab während der Operation relativ viel Raum erforderlich, wobei es infolge der Krümmung der Wirbelsäule und der geneigt zur Längsachse der Wirbelkörper eingesetzten Pedikelschrauben möglich ist, daß die bereits plazierten Pedikelschrauben die Plazierung der Pedikelschrauben am unmittelbar benachbarten Wirbel behindern oder sogar verhindern.

Pedikelschrauben der eingangs genannten Art sind in der WO 99/65415 (Basis für den Oberbegriff des Anspruchs 1) offenbart. Bei diesen Pedikelschrauben ist das Kopfteil kugelförmig gestaltet an dem dem Gewindeschaft abgewandten freien Ende angeordnet. Die Pedikelschraube wird von oben mit ihrem Gewindeschaft durch eine Bohrung des Bügelteils gesteckt, wobei das Kopfteil in einer Kopfteilaufnahme positioniert wird, von der seitlich Stege emporragen, die auf den einander zugewandten Seiten senkrecht zur Gewindeachse verlaufende Nuten aufweisen, in die nach dem Einlegen des Stabes ein Sicherungsglied eingeschoben werden kann. Das Bügelteil ist über die Kopfteilaufnahme ausschließlich um die Gewindeachse drehbar, so daß eine Anpassung der Lage des Bügelteils an die Position des Stabes nur sehr begrenzt möglich ist.

Die EP 0 346 521 A1 zeigt eine Pedikelschraube, bei der das Kopfteil einstückig mit dem nach oben geöffneten Bügelteil ausgebildet ist. Die Sicherung des Stabes in der Aufnahme erfolgt durch ein Verschlußstück, das auf die Öffnung des Bügelteils aufgeklipst wird. Eine Justiermöglichkeit der Pedikelschraube gegenüber dem Stab fehlt.

Der Erfindung liegt daher die Aufgabe zugrunde, eine Pedikelschraube der eingangs genannten Art so auszubilden, daß die Fixierung des Stabes mit der Pedikelschraube erleichtert wird.

Diese Aufgabe wird nach der Erfindung bei einer Pedikelschraube der eingangs genannten Art dadurch gelöst, daß das Kopfteil weiter eine drehbar auf dem Rundstück gelagerte, polyaxial verdrehbare Kappe aufweist, und daß das Bügelteil durch eine in axialer Richtung erfolgende Steckbewegung in einem Rastsitz an der Kappe fixierbar ist.

Die Erfindung bietet den Vorteil, daß nach dem Plazieren der Pedikelschrauben in dem Wirbelkörper und der Positionierung des Stabes die weiteren erforderlichen Maßnahmen des Operateurs nur in der Längsrichtung des Gewindeschaftes durchgeführt werden müssen, also gegenüber dem Raum der ohnehin für das Einschrauben der Pedikelschrauben benötigt wird, kein zusätzlicher Raum erforderlich ist. Dabei ist eine individuelle Anpassung an den Verlauf des in der Regel gekrümmten Stabes möglich, da neben der geeigneten Wahl der Drehlage des Gewindeschaftes der Pedikelschraube mit dem Kopfteil zusätzlich die Kappe um das Rundstück verdreht werden kann.

Die so gegebene Verdrehbarkeit unterliegt den geringsten Einschränkungen, wenn das Rundstück als Halbkugel mit senkrecht zu dem Gewindeschaft orientierter, zum freien Ende weisender Planfläche ausgebildet ist.

Ist zusätzlich auf der Planfläche eine Kalotte mit gegenüber der Halbkugel geringeren Durchmessers angeordnet, besteht die Möglichkeit, den Stab an einem Auflagepunkt unmittelbar durch das Rundstück abzustützen, wobei die Drehbarkeit infolge des kleinen Durchmessers der Kalotte für den Stab relativ zum Rundstück nicht beeinträchtigt ist.

Nach einer besonders bevorzugten Ausführungsform der Erfindung ist vorgesehen, daß an den freien Enden der Seitenschenkel Rastnasen ausgebildet sind, die zur Ausbildung des Rastsitzes bei der Steckbewegung an dem Kappe einhaken.

Um eine ungewollte Verdrehung der Kappe zu verhindern, besteht die Möglichkeit, Mittel zur Blockade der Drehbarkeit von der Kappe auf dem Rundstück vorzusehen. Diese sind zweckmäßigerweise so gestaltet, daß die einander zugewandten Oberflächen von dem Rundstück und der Kappe eine Oberflächenprofilierung aufweisen, und daß in der Grundplatte des Bügelteils eine Gewindebohrung ausgebildet ist für eine der Fixierung des Stabes dienende Sicherungsschraube. Sobald beim Einschrauben der Sicherungsschraube diese an dem Stab zu Anlage kommt, wird der Stab gegen das Rundstück gepreßt, während die Sicherungsschraube sich an dem Stab abstützt und damit auch das Bügelteil gegen die Kappe verspannt, die somit gleichfalls an der dem Stab gegenüberliegenden Seite an das Rundstück gepreßt wird, was mit der geeigneten Oberflächenprofilierung ausgenutzt wird, die Drehbarkeit zu beschränken.

Um einen genau definierten Sitz des Bügelteiles auf der Kappe dauerhaft zu gewährleisten, ist die Pedikelschraube so gestaltet, daß an der Kappe Endplatten mit jeweils einem in axialer Richtung verlaufenden Führungssteg angeordnet sind, und daß jeder Seitenschenkel eine Führungsnut für den Führunssteg aufweist. Alternativ besteht zum Erreichen dieses Zieles natürlich auch die Möglichkeit, daß die Führungsstege und die Führungsnuten an den Endplatten und den Seitenschenkeln miteinander vertauscht sind.

Zur Vermeidung einer Aufspreizung der Seitenschenkel bei in axialer Richtung auf das Bügelteil einwirkenden Kräften ist vorgesehen, daß die Rastnasen die freien Kanten der Endplatten hintergreifen.

Im Hinblick auf eine möglichst großflächig verteilte Krafteinleitung ist es günstig, wenn die Kappe eine Rinne als Auflager für den Stab aufweist. Dabei ist zur unmittelbaren Übertragung von auf den Stab einwirkenden Kräfte auf die Pedikelschraube bzw die Kalotte in der Rinne eine Öffnung ausgebildet.

Um den Stab in Umfangsrichtung möglichst weit umfassen zu können, ist in der Grundplatte des Bügelteils eine Rinne ausgebildet.

Das Aufstecken des Bügelteils auf die Kappe, zu dem eine leichte Aufspreizung der Seitenschenkel erforderlich ist, wird dadurch erleichtert, daß zwischen der Grundplatte und den Seitenschenkeln eine Nut ausgebildet ist.

Die erfindungsgemäße Pedikelschraube ist weiterhin so gestaltet, daß das Kopfteil an einer Verjüngung des Gewindeschafts angesetzt ist. Dies bietet den Vorteil, daß die Kappe über einen größeren Winkelbereich auf dem Rundstück verstellt werden kann, ohne daß die Kappe an dem Gewindeschaft zur Anlage kommt.

Im folgenden wird die Erfindung an einem in der Zeichnung dargestellten Ausführungsbeispiel näher erläutert; es zeigen:
- Fig. 1: eine perspektivische Darstellung der erfindungsgemäßen Pedikelschraube mit einem am Kopfteil fixierten Stab,

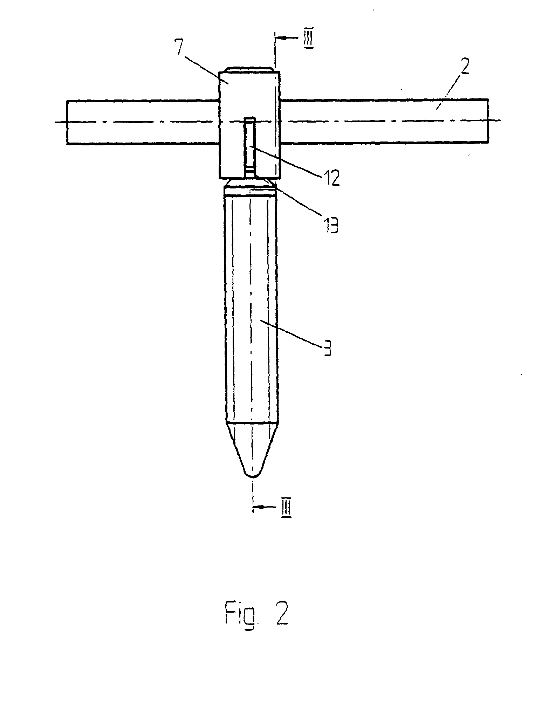

- Fig. 2: eine Seitenansicht der Pedikelschraube,
- Fig. 3: der Schnitt III-III aus Fig. 2,

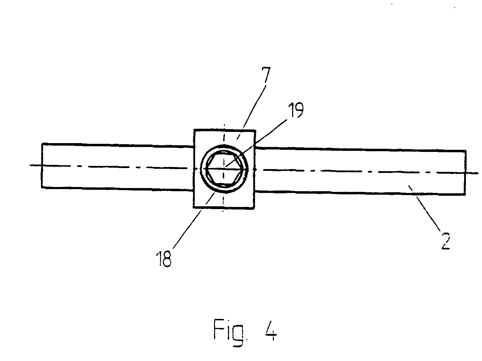

- Fig. 4: eine Draufsicht auf die Pedikelschraube aus Fig. 2,
- Fig. 5: eine Seitenansicht des Gewindeschaftes mit dem Kopfteil,
- Fig. 6: eine perspektivische Darstellung der auf das Rundstück aufgesetzten Kappe,
- Fig. 7: eine weitere perspektivische Darstellung der Pedikelschraube aus Fig. 6 aus einem anderen Blickwinkel,
- Fig. 8: der Schnitt VIII-VIII aus Fig. 7,
- Fig. 9: der Schnitt IX-IX aus Fig. 7
- Fig. 10: eine der Fig. 9 entsprechende Darstellung mit einer gegenüber Fig. 9 verdrehten Kappe, und
- Fig. 11: eine perspektivischen Darstellung des U-förmigen Bügelteils.

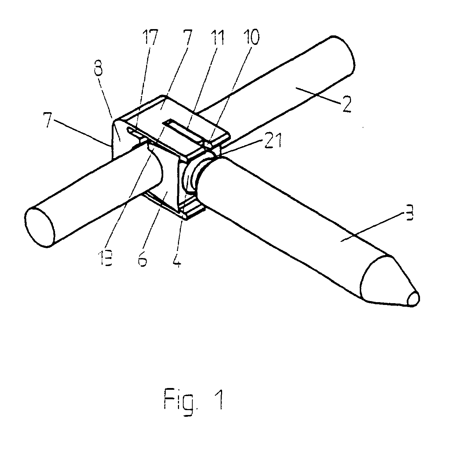

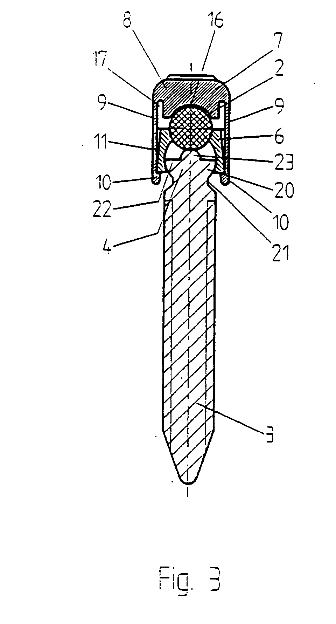

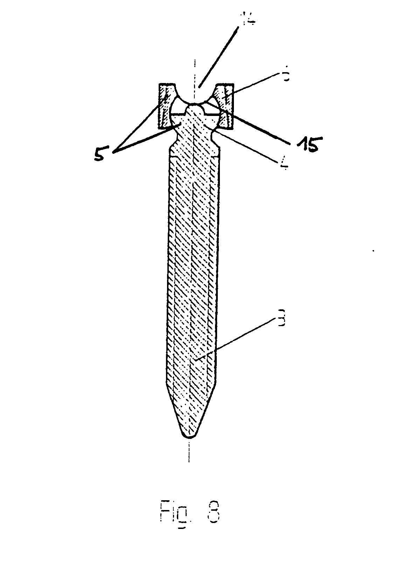

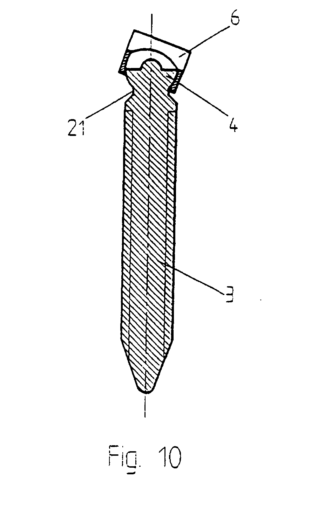

Die in der Fig. 1 dargestellte Pedikelschraube 1 wird benutzt im Zusammenhang mit Implantaten, die der Korrektur und Stabilisierung der Wirbelsäule dienen und die dazu einen an der Wirbelsäule entlanggeführten Stab 2 verwenden. Die Pedikelschraube 1 besteht aus einem Gewindeschaft 3, an dessen einem axialen Ende ein durch ein Rundstück 4 und eine Kappe 6 gebildetes Kopfteil 5 angeordnet ist. Dabei ist das Rundstück 4 als Halbkugel 20 ausgebildet, deren Planfläche 22 senkrecht zu dem Gewindeschaft 3 orientiert ist und nach außen weist. Auf der Planfläche 22 der Halbkugel 20 ist eine Kalotte 23 angeordnet, deren Durchmesser kleiner als der der Halbkugel 20 ist. Aus diesem Aufbau ergibt sich, daß die Kappe 6 polyaxial um das Rundstück 4 verdrehbar und eine gute Anpassungsmöglichkeit an die Lage des an der Wirbelsäule entlanggeführten Stabes 2 gegeben ist (Fig. 3,8,10).

Die auf dem Rundstück 4 angeordnete Kappe 6 wird von einem U-förmigen Bügelteil 7 übergriffen, das eine Aufnahme für den Stab 2 bereitstellt. Das Bügelteil 7 besteht aus einer Grundplatte 8 und zwei Seitenschenkeln 9, an deren freien Enden Rastnasen 10 ausgebildet sind. Zur Fixierung des Bügelteils 7 wird dieses durch eine ausschließlich in axialer Richtung erfolgende Steckbewegung auf die Kappe 6 aufgesteckt, wobei die Rastnasen 10 an den Kanten von der Kappe 6 zugeordneten Endplatten 11 in einem Rastsitz einhaken. Um eine lagerichtige Verbindung des Bügelteils 7 mit der Kappe 6 zu gewährleisten, sowie um ein Verschieben des Bügelteiles 7 in Längsrichtung des Stabes 2 zu vermeiden, sind an den Endplatten 11 Führungsstege 12 ausgebildet, die mit einer Führungsnut 13 zusammenwirken, die an den Seitenschenkeln 9 des Bügelteils 7 ausgebildet sind. Nach einer selber nicht in der Zeichnung dargestellten Ausführungsform ist es selbstverständlich auch möglich, daß die Führungsstege 12 und die Führungsnuten 13 an den Endplatten 11 und den Seitenschenkeln 9 miteinander vertauscht sind.

Eine in der Grundplatte 8 des Bügelteils 7 angeordnete Gewindebohrung 18 dient zur Aufnahme einer Sicherungsschraube 19, mit der der Stab 2 gegen Verschiebungen in seiner Längsrichtung gesichert werden kann. Diese Sicherungsschraube 19 verspannt im Zusammenwirken mit den Rastnasen 10 auch das Bügelteil 7 gegenüber der Kappe 6, was weiterhin dazu ausgenutzt wird, um die Kappe 6 gegen die Halbkugel 20 zu pressen. Wenn nach einer Ausführungsform die einander zugewandten Oberflächen von dem Rundstück 4 und der Kappe 6 eine Oberflächenprofilierung aufweisen, wirkt dies als Mittel zur Blockade der Drehbarkeit von der Kappe 6 und dem Rundstück 4, wobei die Gestaltung der Oberflächenprofilierung die Möglichkeit offenläßt, die Drehbarkeit vollständig, hinsichtlich mehrerer Achsen oder nur hinsichtlich einer Achse zu unterbinden.

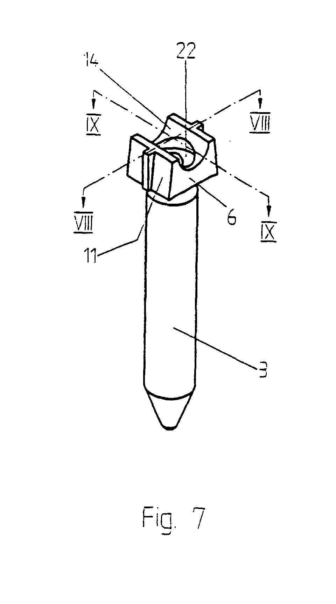

Wie insbesondere aus Fig. 7 ersichtlich ist, weist die Kappe 6 eine Rinne 14 als Auflager für den Stab 2 auf. Die Umfassung des Stabes 2 durch die Pedikelschraube 1 wird durch eine in der Grundplatte 8 des Bügelteils 7 ausgebildete Rinne 16 komplettiert.

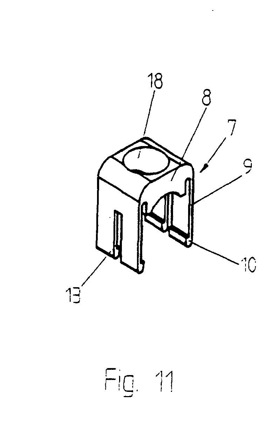

Aus den Fig. 1, 3 und 11 ist ersichtlich, daß am Bügelteil 7 zwischen der Grundplatte 8 und den Seitenschenkeln 9 eine Nut 17 ausgebildet ist, die das Aufspreizen des Bügelteil 7 beim Aufsteckvorgang erleichtert. Ein unerwünschtes Aufspreizen nach dem Aufstecken des Bügelteils 7 wird dadurch verhindert, daß die Rastnasen 10 die freien Kanten der Endplatten 11 hintergreifen.

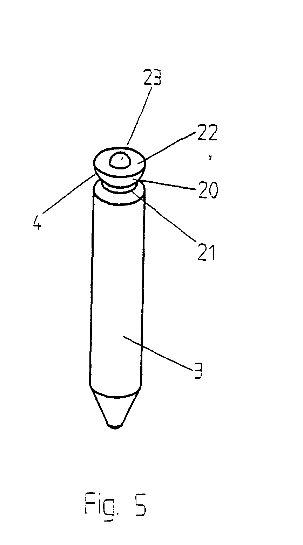

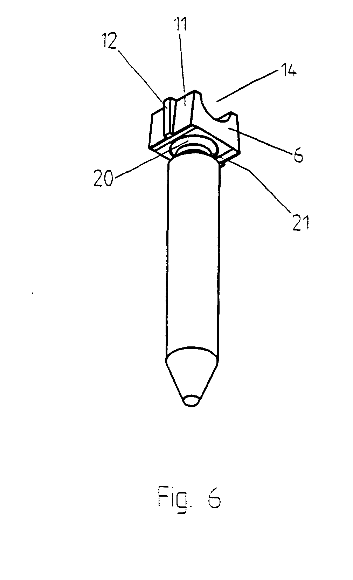

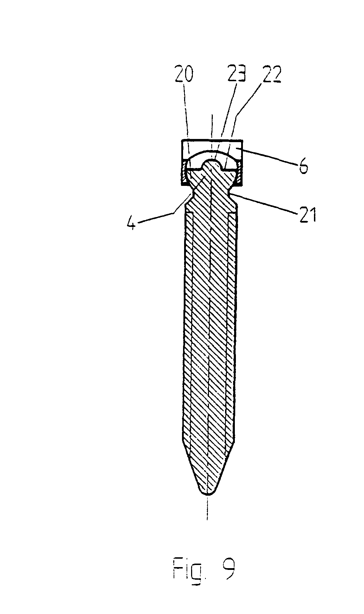

Aus der Zeichnung, insbesondere den Fig. 5 bis 10 ist weiterhin ersichtlich, daß das Kopfteil 5 an einer Verjüngung 21 des Gewindeschaftes 3 angesetzt ist, der Drehwinkel der Kappe 6 also nicht frühzeitig durch dessen Anlage an dem Gewindeschaft 3 begrenzt ist.

## Patentansprüche

1. Pedikelschraube für Implantate zur Korrektur und Stabilisierung der Wirbelsäule, mit einem an dem axialen Ende eines Gewindeschafts (3) angeordneten Kopfteil (5), und mit einem Bügelteil (7), das an dem Kopfteil anschließbar ist, das U-förmig mit einer Grundplatte (8) und zwei Seitenschenkeln (9) gebildet ist und eine Aufnahme für einen am Kopfteil (5) fixierbaren Stab (2) aufweist, wobei das Kopfteil (5) ein Rundstück (4) aufweist, **dadurch gekennzeichnet, daß** das Kopfteil (5) weiter eine drehbar auf dem Rundstück (4) gelagerte, polyaxial verdrehbare Kappe (6) aufweist, und daß das Bügelteil (7) durch eine in axialer Richtung erfolgende Steckbewegung in einem Rastsitz an der Kappe (6) fixierbar ist.

2. Pedikelschraube nach Anspruch 1, **dadurch gekennzeichnet, daß** das Rundstück (4) als Halbkugel (20) mit senkrecht zu dem Gewindeschaft (3) orientierter, zum freien Ende weisender Planfläche (22) ausgebildet ist.

3. Pedikelschraube nach Anspruch 2, **dadurch gekennzeichnet, daß** auf der Planfläche (22) eine Kalotte (23) mit gegenüber der Halbkugel (20) geringerem Durchmesser angeordnet ist.

4. Pedikelschraube nach einem der Ansprüche 1 bis 3, **dadurch gekennzeichnet, daß** an den freien Enden der Seitenschenkel (9) Rastnasen (10) ausgebildet sind, die zur Ausbildung des Rastsitzes bei der Steckbewegung an der Kappe (6) einhaken.

5. Pedikelschraube nach einem der Ansprüche 1 bis 4, **dadurch gekennzeichnet, daß** Mittel zur Blockade der Drehbarkeit der Kappe (6) auf dem Rundstück (4) vorgesehen sind.

6. Pedikelschraube nach Anspruch 5, **dadurch gekennzeichnet, daß** die einander zugewandten Oberflächen von dem Rundstück (4) und der Kappe (6) eine Oberflächenprofilierung aufweisen, und daß in der Grundplatte (8) des Bügelteils (7) eine Gewindebohrung (18) ausgebildet ist für eine der Fixierung des Stabes (2) dienende Sicherungsschraube (19) .

7. Pedikelschraube nach einem der Ansprüche 1 bis 6, **dadurch gekennzeichnet, daß** an der Kappe (6) Endplatten (11) mit jeweils einem in axialer Richtung verlaufenden Führungssteg (12) angeordnet sind, und daß jeder Seitenschenkel (9) eine Führungsnut (13) für den Führungssteg (12) aufweist.

8. Pedikelschraube nach Anspruch 7, **dadurch gekennzeichnet, daß** die Führungsstege (12) und die Führungsnuten (13) an den Endplatten (11) und den Seitenschenkeln (9) miteinander vertauscht sind.

9. Pedikelschraube nach einem der Ansprüche 4 bis 8, **dadurch gekennzeichnet, daß** die Rastnasen (10) die freien Kanten der Endplatten (11) hintergreifen.

10. Pedikelschraube nach einem der Ansprüche 1 bis 9, **dadurch gekennzeichnet, daß** die Kappe (6) eine Rinne (14) als Auflager für den Stab (2) aufweist.

11. Pedikelschraube nach Anspruch 10, **dadurch gekennzeichnet, daß** in der Rinne (14) eine Öffnung (15) ausgebildet ist.

12. Pedikelschraube nach einem der Ansprüche 4 bis 11, **dadurch gekennzeichnet, daß** in der Grundplatte (8) des Bügelteils (7) eine Rinne (16) ausgebildet ist.

13. Pedikelschraube nach einem der Ansprüche 2 bis 12, **dadurch gekennzeichnet, daß** zwischen der Grundplatte (8) und den Seitenschenkeln (9) eine Nut (17) ausgebildet ist.

14. Pedikelschraube nach einem der Ansprüche 1 bis 13, **dadurch gekennzeichnet, daß** das Kopfteil (5) an einer Verjüngung (21) des Gewindeschafts (3) angesetzt ist.

## Claims

1. A pedicle screw for implants for correction and stabilisation of the spinal column, comprising a head portion (5) which is arranged at the axial end of a screwthreaded shank (3) and a stirrup portion (7) which can be connected to the head portion and which is of a U-shaped configuration with a base plate (8) and two side limbs (9) and has a receiving means for a rod (2) which can be fixed to the head portion (5), wherein the head portion (5) has a round part (4) **characterised in that** the head portion (5) further has a polyaxially rotatable cap (6) which is mounted rotatably on the round part (4) and that the stirrup portion (7) can be fixed in a retaining fit on the cap (6) by a plug-in movement in the axial direction.

2. A pedicle screw according to claim 1 **characterised in that** the round part (4) is in the form of a hemispherical portion (20) with a flat surface (22) which is oriented in perpendicular relationship to the screwthreaded shank (3) and which faces towards the free end.

3. A pedicle screw according to claim 2 **characterised in that** arranged on the flat surface (22) is a part-spherical portion (23) of a smaller diameter than the hemispherical portion (20).

4. A pedicle screw according to one of claims 1 to 3 **characterised in that** provided at the free ends of the side limbs (9) are retaining noses (10) which come into hooking engagement on the cap (6) to provide the retaining fit in the plug-in movement.

5. A pedicle screw according to one of claims 1 to 4 **characterised in that** there are provided means for blocking the rotatability of the cap (6) on the round part (4).

6. A pedicle screw according to claim 5 **characterised in that** the mutually facing surfaces of the round part (4) and the cap (6) have a surface profiling and that provided in the base plate (8) of the stirrup portion (7) is a screwthreaded bore (18) for a securing screw (19) which serves to fix the rod (2).

7. A pedicle screw according to one of claims 1 to 6 **characterised in that** arranged on the cap (6) are end plates (11) each having a respective guide leg (12) extending in the axial direction and that each side limb (9) has a guide groove (13) for the guide leg (12).

8. A pedicle screw according to claim 7 **characterised in that** the guide legs (12) and the guide grooves (13) on the end plates (11) and the side limbs (9) are interchanged with each other.

9. A pedicle screw according to one of claims 4 to 8 **characterised in that** the retaining noses (10) engage behind the free edges of the end plates (11).

10. A pedicle screw according to one of claims 1 to 9 **characterised in that** the cap (6) has a channel (14) as a support for the rod (2).

11. A pedicle screw according to claim 10 **characterised in that** an opening (15) is provided in the channel (14).

12. A pedicle screw according to one of claims 4 to 11 **characterised in that** a channel (16) is provided in the base plate (8) of the stirrup portion (7).

13. A pedicle screw according to one of claims 2 to 12 **characterised in that** a groove (17) is provided between the base plate (8) and the side limbs (9).

14. A pedicle screw according to one of claims 1 to 13 **characterised in that** the head portion (5) is attached to a constriction (21) of the screwthreaded shank (3).

## Revendications

1. Vis pédiculaire pour implants de correction et de stabilisation de la colonne vertébrale, comportant une partie tête (5) qui est disposée à l'extrémité axiale d'une tige filetée (3) et une partie formant étrier (7) qui peut être liée à la partie tête, est conformée en U, avec une plaque de base (8) et deux ailes latérales (9), et présente un logement pour une tige (2) à fixer à la partie tête (5), la partie tête (5) comportant une portion ronde (4), **caractérisée par le fait que** la partie tête (5) comporte en outre une coiffe (6) tournante polyaxe, qui est montée avec possibilité de rotation sur la portion ronde et **par le fait que** la partie formant étrier (7) peut être fixée par encliquetage à la coiffe (6), par un mouvement d'enfichage dans la direction axiale.

2. Vis pédiculaire selon la revendication 1, **caractérisée par le fait que** la portion ronde (4) est conformée en demi-sphère (20) avec une surface plane (22) orientée perpendiculairement à la tige filetée (3) et tournée vers l'extrémité libre.

3. Vis pédiculaire selon la revendication 2, **caractérisée par le fait qu'**une calotte (23) de diamètre plus faible par rapport à la demi-sphère (20) est disposée sur la surface plane (22).

4. Vis pédiculaire selon une des revendications 1 à 3, **caractérisée par le fait que** des nez d'encliquetage (10) sont aménagés aux extrémités libres des ailes latérales (9), lesquels nez d'encliquetage pour former la liaison par encliquetage s'accrochent sur la coiffe (6) lors du mouvement d'enfichage.

5. Vis pédiculaire selon une des revendications 1 à 4, **caractérisée en ce que** des moyens sont prévus pour bloquer la capacité de rotation de la coiffe (6) sur la portion ronde (4).

6. Vis pédiculaire selon la revendication 5, **caractérisée en ce que** les faces en regard de la portion ronde (4) et de la coiffe (6) ont leurs surfaces profilées, et dans la plaque de base (8) de la partie formant étrier (7) se trouve un alésage fileté (8) pour accueillir une vis de sécurité (19) servant à assurer la fixation de la tige (2).

7. Vis pédiculaire selon une des revendications 1 à 6, **caractérisée en ce que** la coiffe (6) comporte des plaques d'extrémité (11) portant chacune une barrette de guidage (12) dirigée axialement, tandis que chaque aile latérale (9) présente une rainure de guidage (13) pour accueillir la barrette de guidage (12).

8. Vis pédiculaire selon la revendication 7, **caractérisée en ce que** les barrettes de guidage (12) et les rainures de guidage (13) sur les plaques d'extrémité (11) et sur les ailes latérales (9) peuvent être échangées entre elles.

9. Vis pédiculaire selon une des revendications 4 à 8, **caractérisée en ce que** les nez d'encliquetage (10) sont en prise par-dessous avec les bords libres des plaques d'extrémité (11).

10. Vis pédiculaire selon une des revendications 1 à 9, **caractérisée en ce que** la coiffe (6) présente une rainure (14) servant d'appui à la tige (2).

11. Vis pédiculaire selon la revendication 10, **caractérisée en ce qu'**une ouverture (15) est prévue dans la rainure (14).

12. Vis pédiculaire selon une des revendications 4 à 11, **caractérisée en ce que** dans la plaque de base (8) de la partie formant étrier (7) se trouve une rainure (16).

13. Vis pédiculaire selon une des revendications 2 à 12, **caractérisée en ce qu'**entre la plaque de base (8) et chacune des ailes latérales se trouve une rainure (17).

14. Vis pédiculaire selon une des revendications 1 à 13, **caractérisée en ce que** la partie tête (5) fait suite à une partie rétrécie (21) de la tige filetée (3).
